# EUROPEAN PATENT APPLICATION

(11) **EP 4 542 571 A1**
(43) Date of publication of application: **23.04.2025**
(21) Application number: 23839932.3
(22) Date of filing: 11.07.2023
(51) Int. Cl.: G16H 50/20, G16H 40/20, A61B 5/0245, A61B 5/024, G06N 3/04

(54) **METHOD, PROGRAM, AND DEVICE FOR PROVIDING TRANSPORT SERVICES FOR EMERGENCY PATIENT AND MONITORING OF HOSPITALIZED PATIENT ON BASIS OF ELECTROCARDIOGRAMS**

(30) Priority: 14.07.2022 KR 20220086810; 10.07.2023 KR 20230089032
(71) Applicant: Medical AI Co., Ltd., Seoul 06180 (KR)
(72) Inventor: KWON, Joonmyoung, Seoul 06180 (KR); KANG, Seonmi, Seoul 06180 (KR); LEE, Byeongtak, Seoul 06180 (KR); LIM, Seonyu, Seoul 06180 (KR)
(74) Representative: Patentanwaltskanzlei Matschnig & Forsthuber OG
(86) International application number: PCT/KR2023/009864
(87) International publication number: WO 2024/014840

(57) **Abstract**

The present disclosure is directed to a method, program, and device for providing a transport service for an emergency patient and the monitoring of a hospitalized patient based on electrocardiograms. The method includes: obtaining the electrocardiogram data of an emergency patient at preset time intervals; calculating a prediction value corresponding to the likelihood of occurrence of each of a plurality of diseases based on the electrocardiogram data by using a pre-trained neural network model; computing the level of severity for the classification of the emergency patient based on the prediction value; and selecting a target institution or facility suitable for the treatment of the emergency patient by using the computed level of severity.

## Description

### Technical Field

The present disclosure relates to a method for providing a transport service for an emergency patient and the monitoring of a hospitalized patient based on electrocardiograms, and more specifically, to a method that predicts the likelihood of occurrence of a heart disease based on electrocardiogram data by using a pre-trained neural network model and transports an emergency patient to an optimal target institution or facility for treatment based on emergency symptoms.

### Background Art

The emergency medical system can be basically divided into pre-hospital emergency treatment and in-hospital emergency treatment. In particular, pre-hospital emergency treatment is performed through reporting, rescue, on-site treatment and emergency patient transport stages from the occurrence of an emergency patient, and includes not only the securement of an ambulance that can rapidly be dispatched and transport a patient sufficiently within a set time but also appropriate emergency treatment in the ambulance. Next, in-hospital emergency treatment includes emergency room treatment, hospitalization treatment, and treatment at an emergency medical institution.

In Korea, the fire department 119 pre-hospital emergency medical system is intended to provide specialized emergency treatment by 119 emergency medical technicians (or paramedics) to improve the survival rate and provide emergency medical services based on patient-centered prevention and management on site. In this case, 119 emergency medical technicians measure an electrocardiogram (ECG), oxygen saturation (Spo2), body temperature, blood pressure, etc. to check a patient's condition. Among them, the electrocardiogram has great clinical importance in diagnosing cardiovascular diseases, so that the 119 emergency medical technicians are equipped with an electrocardiogram measurement device, an electrocardiogram monitor (for patient monitoring), and an advanced defibrillator, which are equipment related to electrocardiogram measurement.

Recently, in Korea, due to the aging of the population, rapid increase in cardiovascular diseases, and increase in various accidents, the number of patients having pre-hospital cardiac arrest has been increasing. However, according to the results of various domestic studies, the survival rate of patients having prehospital cardiac arrest is 2 to 17%, which is lower than in the West and has not changed much compared to the past. According to the emergency medical report of the Korea Health Industry Development Institute, it is found that when initial emergency treatment is performed appropriately, the survival rate of emergency patients improves from 20% to 50%.

Unlike in the case of general patients, in the case of emergency patients, emergency treatment within 30 minutes of the onset of illness or injury has a significant impact on a patient's life. Accordingly, emergency treatment should begin at the scene of illness or injury, and treatment should not be interrupted during transport. Even after arriving at a hospital, intensive treatment by medical staff from various fields should be performed rapidly.

In particular, in the event of an emergency related to heart disease, it is necessary to rapidly transport an emergency patient to a hospital where treatment can be performed through an accurate diagnosis at a pre-hospital emergency treatment stage. However, among emergency patients related to heart disease, myocardial infarction patients need to undergo percutaneous coronary intervention (PCI) within 2 hours. However, there is no way to immediately know which hospital can perform PCI at the prehospital emergency treatment stage.

When a 119 ambulance transports a myocardial infarction patient to a hospital that cannot perform PCI, the 119 ambulance cannot be used for transfer between hospitals, so that a private ambulance needs to be used, which may delay the transfer process for the emergency patient for a long time.

Furthermore, even when an emergency patient diagnosed with myocardial infarction boards a 119 ambulance, a 119 emergency medical technician needs to call several hospitals, to which the emergency patient can be transferred, to select a hospital where PCI can be performed, with the result that a problem arises in that emergency treatment time is delayed or appropriate treatment is not performed during this communication process.

Above all, when 119 emergency medical technicians have difficulty distinguishing between patients having simple chest pain and patients having myocardial infarction based on the results of electrocardiogram measurements, and thus cannot distinguish between patients who definitely require PCI and patients who do not require PCI and transfer all patients, including patients having simple chest pain, to a hospital where PCI can be performed, the efficiency of emergency treatment will decrease, and also emergency medical resources such as transport time and preparation for PCI will also be wasted, which may actually cause problems in the appropriate treatment of patients having myocardial infarction.

### Disclosure

### Technical Problem

The present disclosure has been conceived in response to the above-described background art, and is directed to the provision of a method that predicts the likelihood of occurrence of a heart disease based on electrocardiogram data by using a pre-trained neural network model and transports an emergency patient to an optimal target institution or facility for treatment based on emergency symptoms.

However, the objects to be accomplished by the present disclosure are not limited to the object mentioned above, and other objects not mentioned may be clearly understood based on the following description.

### Technical Solution

According to one embodiment of the present disclosure for achieving the above-described object, there is disclosed a method for providing a transport service for an emergency patient and the monitoring of a hospitalized patient based on electrocardiograms. The method may include: obtaining the electrocardiogram data of an emergency patient at preset time intervals; calculating a prediction value corresponding to the likelihood of occurrence of each of a plurality of diseases based on the electrocardiogram data by using a pre-trained neural network model; computing the level of severity for the classification of the emergency patient based on the prediction value; and selecting a target institution or facility suitable for the treatment of the emergency patient by using the computed level of severity.

Alternatively, computing the level of severity for the classification of the emergency patient based on the prediction value may include identifying the trend of changes in the prediction value and determining the level of severity based on the identified trend of changes and the characteristics of the plurality of diseases.

Alternatively, identifying the trend of changes in the prediction value and determining the level of severity based on the identified trend of changes and the characteristics of the plurality of diseases may include: selecting one of determination methods for identifying the trend of changes in the prediction value according to the characteristics of the plurality of diseases; and determining the level of severity based on the identified trend of changes identified according to the selected determination method.

Alternatively, the determination method may be any one of the method of determining whether the prediction value computed by the neural network model is in an upward trend, the method of determining whether the prediction value is in a downward trend or repeatedly falls and rises, the method of making a determination using the slope of a rising curve for the prediction value, the method of determining whether the prediction value deviates from a predetermined cutoff, and the method of determining whether the prediction value deviates from a predetermined size by marking the prediction value on a graph and then computing the size of an area.

Alternatively, selecting the target institution or facility suitable for treatment of the emergency patient by using the computed level of severity may include: classifying the emergency patient as either a first patient who requires a specific procedure or a second patient who does not require a specific procedure by using the level of severity of the emergency patient measured by severity classification used in common by emergency personnel and medical staff and the level of severity computed based on the prediction value; extracting candidate hospitals for the treatment of the emergency patient from a group of hospitals matching the classification based on location and traffic information including at least one of the location of the emergency patient, real-time traffic conditions, and the number of ambulances heading to each hospital; and computing the degrees of suitability of the candidate hospitals by using capacity information including at least one of the emergency room congestion, emergency facility information, and medical staff information of each of the candidate hospitals, and setting up a transport plan according to the order of priority of the hospitals based on the computed degrees of suitability.

Alternatively, the first patient may include at least one of a patient who requires a procedure that can only be performed at a specific hospital or by specific medical staff and a patient whose level of severity computed based on the prediction value is determined to be higher than a preset reference for each of the diseases.

Alternatively, extracting the candidate hospitals for the treatment of the emergency patient from the group of hospitals matching the classification based on the location and traffic information including at least one of the location of the emergency patient, the real-time traffic conditions, and the number of ambulances heading to each hospital may include: computing expected transport time information for the hospitals included in the hospital group matching the classification; and extracting candidate hospitals from the hospital group matching the classification based on the expected transport time information.

Alternatively, computing the degrees of suitability of the candidate hospitals by using the capacity information including at least one of the emergency room congestion, emergency facility information, and medical staff information of each of the candidate hospitals, and setting up the transport plan according to the order of priority of the hospitals based on the computed degrees of suitability may include providing a communication service in order to inquire of N hospitals, selected according to the order of priority of the hospitals, about whether to accept the patient.

According to another embodiment of the present disclosure, there is provided a computer program stored in a computer-readable storage medium, the computer program performing operations for providing a transport service for an emergency patient and the monitoring of a hospitalized patient based on electrocardiograms when executed on one or more processors, wherein the operations includes operations of: obtaining the electrocardiogram data of an emergency patient at preset time intervals; calculating a prediction value corresponding to the likelihood of occurrence of each of a plurality of diseases based on the electrocardiogram data by using a pre-trained neural network model; computing the level of severity for the classification of the emergency patient based on the prediction value; and selecting a target institution or facility suitable for the treatment of the emergency patient by using the computed level of severity.

Meanwhile, according to one embodiment of the present disclosure, there is provided a computing device for providing a transport service for an emergency patient and the monitoring of a hospitalized patient based on electrocardiograms, the computing device including: a processor including at least one core; and memory including program codes executable on the processor; wherein the processor, according to execution of the program codes, obtains the electrocardiogram data of an emergency patient at preset time intervals; calculates a prediction value corresponding to the likelihood of occurrence of each of a plurality of diseases based on the electrocardiogram data by using a pre-trained neural network model; computes the level of severity for the classification of the emergency patient based on the prediction value; and selects a target institution or facility suitable for the treatment of the emergency patient by using the computed level of severity.

### Advantageous Effects

The method for providing a transport service for an emergency patient and the monitoring of a hospitalized patient based on electrocardiograms according to one embodiment of the present disclosure may rapidly and accurately predict the likelihood of occurrence of a heart disease based on electrocardiogram data by using the pre-trained neural network model, may identify the level of severity of an emergency patient based on the predicted trend of changes in the likelihood of occurrence of a heart disease and the characteristics of a plurality of diseases and then classify a patient requiring a specific procedure based on the identified level of severity, and may transport the emergency patient to an optimal target institution or facility for treatment for emergency symptoms of the patient classified in this manner, thereby further increasing the appropriateness of emergency treatment for emergency symptoms.

### Description of Drawings

FIG. 1 is a block diagram of a computing device according to one embodiment of the present disclosure;
FIG. 2 is a block diagram illustrating the configuration of a system for providing a transport service for an emergency patient and the monitoring of a hospitalized patient based on electrocardiograms according to one embodiment of the present disclosure;
FIG. 3 is a diagram illustrating a process of identifying the trend of changes using a neural network model according to one embodiment of the present disclosure;
FIG. 4 is a flowchart illustrating a method for providing a transport service for an emergency patient and the monitoring of a hospitalized patient based on electrocardiograms according to one embodiment of the present disclosure; and
FIG. 5 is a flowchart illustrating in detail a method of selecting a target institution or facility suitable for the treatment of an emergency patient according to one embodiment of the present disclosure.

### Mode for Invention

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "nth (n is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "obtainment" used herein can be understood to mean not only receiving data over a wired/wireless communication network connecting with an external device or a system, but also generating data in an on-device manner.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

The term "block" used herein may be understood as a set of components that are classified based on various criteria such as type, function, etc. Accordingly, the components classified as one "block" may be changed in various manners depending on the criterion. For example, a neural network "block" may be understood as a set of neural networks that include one or more neural networks. In this case, it may be assumed that the neural networks included in the neural network "block" perform specific operations in the same manner. The foregoing descriptions of the terms are intended to help to understand the present disclosure. Accordingly, it should be noted that unless the above-described terms are explicitly described as limiting the content of the present disclosure, the terms in the content of the present disclosure are not used in the sense of limiting the technical spirit of the present disclosure.

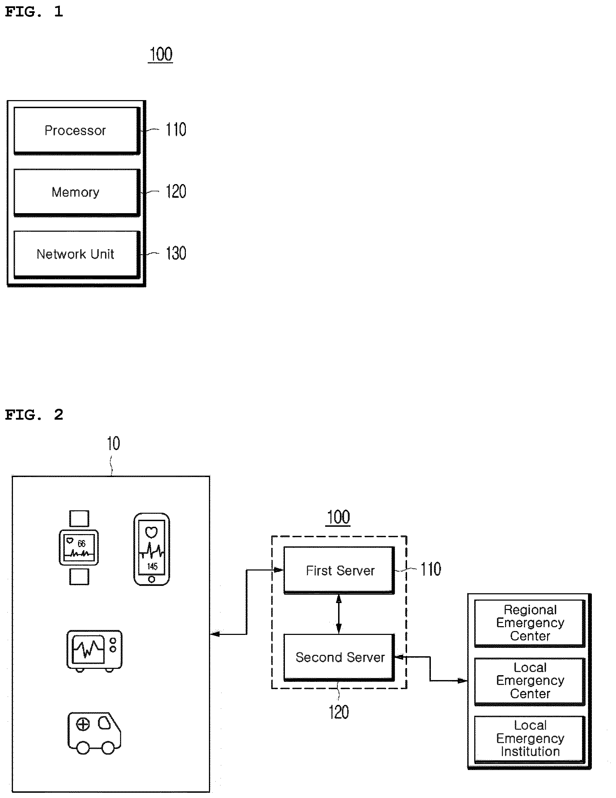

FIG. 1 is a block diagram of a computing device according to one embodiment of the present disclosure.

A computing device 100 according to one embodiment of the present disclosure may be a hardware device or part of a hardware device that performs the comprehensive processing and calculation of data, or may be a software-based computing environment that is connected to a communication network. For example, the computing device 100 may be a server that performs an intensive data processing function and shares resources, or may be a client that shares resources through interaction with a server. Furthermore, the computing device 100 may be a cloud system in which a plurality of servers and clients interact with each other and comprehensively process data. Since the above descriptions are only examples related to the type of computing device 100, the type of computing device 100 may be configured in various manners within a range understandable to those skilled in the art based on the content of the present disclosure.

Referring to FIG. 1, the computing device 100 according to one embodiment of the present disclosure may include a processor 110, memory 120, and a network unit 130. However, FIG. 1 shows only an example, and the computing device 100 may include other components for implementing a computing environment. Furthermore, only some of the components disclosed above may be included in the computing device 100.

The processor 110 according to one embodiment of the present disclosure may be understood as a constituent unit including hardware and/or software for performing computing operation. For example, the processor 110 may read a computer program and perform data processing for machine learning. The processor 110 may process computational processes such as the processing of input data for machine learning, the extraction of features for machine learning, and the calculation of errors based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within a range understandable to those skilled in the art based on the content of the present disclosure.

The processor 110 may use a pre-trained neural network model to diagnose and predict a disease based on the obtained electrocardiogram data of an emergency patient and enable the emergency patient to be transported to an optimal hospital in the event of an emergency. In this case, the processor 110 may extract electrocardiogram features based on the electrocardiogram data, and may train the neural network model that calculates a prediction value corresponding to the likelihood of occurrence of each of a plurality of heart diseases based on the extracted electrocardiogram features. For example, the processor 110 may train the neural network model to estimate a heart disease by analyzing electrocardiograms based on biological information including information such as gender, age, weight, height, and/or the like together with the electrocardiogram data.

More specifically, the processor 110 may train the neural network model by inputting electrocardiogram data and various types of biological information to the neural network model so that the neural network model can detect changes in electrocardiograms attributable to arrhythmia or another heart disease. In this case, the neural network model may be trained based on an electrocardiogram dataset including the electrocardiogram features extracted from electrocardiogram data and diagnostic data for arrhythmia and other heart diseases. During the process of training the neural network model, the processor 110 may perform the operation of representing at least one neural network block included in the neural network model.

The processor 110 may use the neural network model generated through the above-described training process to extract electrocardiogram features from the electrocardiogram data obtained from an electrocardiogram measurement device and estimate electrocardiogram reading data based on the extracted electrocardiogram features. The processor 110 may generate inference data representing the result of estimating the likelihood of a heart disease by inputting electrocardiogram data and biological information including information such as gender, age, weight, height, and/or the like to the neural network model trained through the above-described process. For example, the processor 110 may provide prediction values such as the presence or absence of arrhythmia or another heart disease, the degree of progression thereof, and/or the like by inputting electrocardiogram data to the trained neural network model.

In addition to the examples described above, the types of medical data and the output of the neural network model may be configured in various manners within a range that will be understandable to those skilled in the art based on the contents of the present disclosure.

The memory 120 according to one embodiment of the present disclosure may be understood as a configuration unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize, and manage data necessary for the processor 110 to perform computation, the combination of data, and program codes executable on the processor 110. For example, the memory 120 may store electrocardiogram data received through the network unit 130, which will be described later. The memory 120 may store program codes configured to operate the neural network model to receive medical data and perform learning, program codes configured to operate the neural network model to receive medical data and perform inference in accordance with the purpose of use of the computing device 100, processed data generated as program codes are executed, etc.

The network unit 130 according to one embodiment of the present disclosure may be understood as a configuration unit that transmits and receives data through any type of known wired/wireless communication system. For example, the network unit 130 may perform data transmission and reception using a wired/wireless communication system such as a local area network (LAN), a wideband code division multiple access (WCDMA) network, a long term evolution (LTE) network, the wireless broadband Internet (WiBro), a 5th generation mobile communication (5G) network, a ultra wide-band wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive data necessary for the processor 110 to perform computation through wired/wireless communication with any system or client or the like. Furthermore, the network unit 130 may transmit data generated through the computation of the processor 110 through wired/wireless communication with any system or client or the like. For example, the network unit 130 may receive medical data through communication with an electrocardiogram measurement device 10 or the like including a wearable device or the like. The network unit 130 may transmit the output data of the neural network model, intermediate data and processed data obtained from the computation process of the processor 110, etc. through communication with the above-described device.

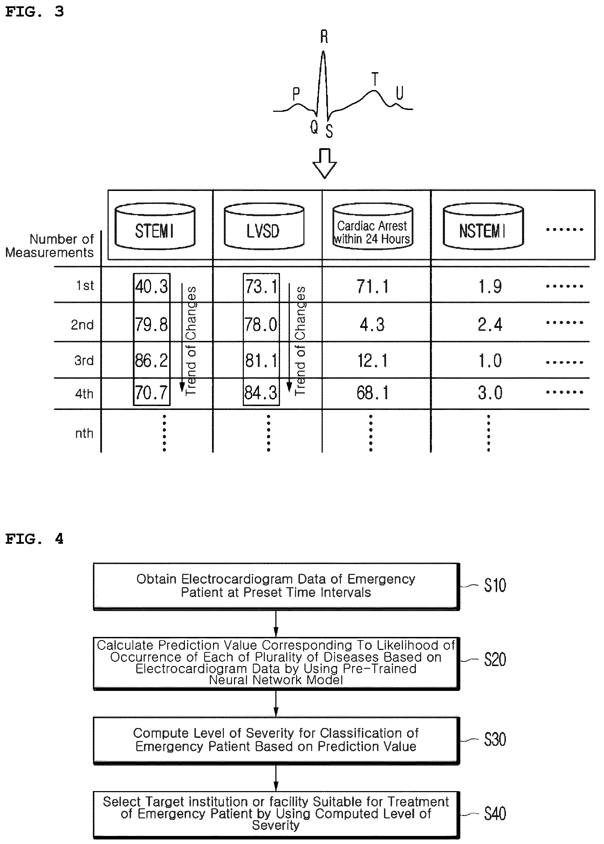

FIG. 2 is a block diagram illustrating the configuration of a system for providing a transport service for an emergency patient and the monitoring of a hospitalized patient based on electrocardiograms according to one embodiment of the present disclosure, and FIG. 3 is a diagram illustrating a process of identifying the trend of changes using a neural network model according to one embodiment of the present disclosure.

The system for providing a transport service for an emergency patient based on electrocardiograms includes, but is not limited to, a local device 10 and a computing device 100.

The local device 10 is a device capable of measuring electrocardiograms, and may be provided for a 119 emergency medical technician, an emergency patient, or an ambulance. Various devices capable of measuring electrocardiograms, such as a wearable device, a portable electrocardiogram measurement device, and a biosignal measurement device, that are worn on the body of an emergency patient and can measure and collect various health indicators, such as heart rate, body fat percentage, blood pressure, and/or the like, may be used as the local device 10. In this case, the local device 10 may measure electrocardiograms using various electrode combinations, such as a single-lead method using not only a wearable device such as a wristwatch or patch but also a 12-lead method, a 6-lead method, and the like. It is also desirable that the electrocardiogram measurement time is increased or decreased and then set according to the signal to be obtained.

The computing device 100 may extract electrocardiogram features including P waves, QRS complexes, and T waves based on the electrocardiogram data, obtained from the electrocardiogram measurement device 10, using a pre-trained neural network model 200. In this case, the neural network model may be trained based on electrocardiogram features including at least one of the frequency of tachycardia, the length of the QT interval, the directions of deviation of the P waves, the R waves, and the T waves, and the duration of the QRS.

This computing device 100 includes a first server 110 for the reading of an electrocardiogram and a second server 120 for the management of an emergency patient. The first server 110 and the second server 120 may be operated in an independent or integrated manner.

As shown in FIG. 3, the first server 110 uses the pre-trained neural network model 200 to calculate a prediction value corresponding to the likelihood of occurrence of each of a plurality of heart diseases based on electrocardiogram data and compute the level of severity for the classification of an emergency patient based on the computed prediction value. The first server 110 may include a classification model by which the pre-trained neural network model 200 can calculate a prediction value corresponding to the likelihood of occurrence of each heart disease. Accordingly, the first server 110 may classify a disease in question as a heart disease with high accuracy by inputting electrocardiogram data to a plurality of pre-trained classification models in stages, and may calculate a prediction value corresponding to the likelihood of occurrence of each heart disease using each classification model.

For example, neural network models may construct a plurality of classification models corresponding to the types of heart diseases to be identified. The classification models may predict the likelihoods of occurrence for respective diseases such as ST elevation myocardial infarction (STEMI), left ventricular systolic dysfunction (LVSD), cardiac arrest within 24 hours, and non-ST segment elevation myocardial infarction (NSTEMI).

In this case, the prediction value provided by each of the classification models may be computed as a numerical value in the range of 0 to 100. The trend of changes in the prediction value of each classification model may be identified, and then the level of severity may be determined based on the identified trend of changes and the characteristics of a plurality of diseases.

The level of severity may be determined by selecting the following determination methods for identifying the trend of changes in the prediction value according to the characteristics of a plurality of diseases, and the levels of severity may be determined based on the trends of changes identified according to the selected determination methods.

That is, the second server 120 selects a determination method based on the characteristics of a disease out of the method of determining whether the prediction value (or the disease score, or the numerical value) computed by the neural network model is in an upward trend, the method of determining whether the prediction value is in a downward trend or repeatedly falls and rises, the method of making a determination using the steepness (e.g., the slope) of a rising curve for the prediction value, the method of determining whether the prediction value deviates from a predetermined cutoff, and the method of determining whether the prediction value deviates from a predetermined size by marking the prediction value on a graph and then computing the size of an area, and finally determines the level of severity.

For example, the levels of severity may be classified into a first level corresponding to a case where the prediction value is in an upward trend, a second level corresponding to a case where the prediction value repeatedly rises and falls without an upward trend under a preset upper limit, a third level corresponding to a case where the prediction value repeatedly rises and falls in the range of the upper limit (e.g., 70) and a preset lower limit (e.g., 30), and a fourth level corresponding to a case where the prediction value repeatedly rises and falls under the lower limit.

The first server 110 sets the level of severity to the third level because the trend of changes in the prediction value repeatedly rises and falls in the range of the upper limit to the lower limit as the number of electrocardiogram measurements increases based on the classification model for the STEMI, and sets the level of severity to the first level because the range of the prediction values increases beyond the upper limit and exhibits an upward trend based on the classification model for the LVSD as the number of electrocardiogram measurements increases. Furthermore, the first server 110 determines that the trend of changes in the prediction value based on the classification model for the cardiac arrest within 24 hours cannot be distinguished as being in an upward or downward trend, and sets the level of severity to the fourth level because the trend of changes in the prediction value based on the classification model for the NSTEMI repeatedly rises and falls under the lower limit.

In this case, when the level of severity is determined to be the fourth level a preset number of times or more, the first server 110 may terminate the prediction value computation using the neural network model 200 and stop the electrocardiogram measurement.

The second server 120 may use the computed level of severity to select a target institution or facility suitable for the treatment of an emergency patient and enable the emergency patient to be transported to the selected target institution or facility. This second server 120 may transfer dispatch information to a fire station or 119 safety center in response to an emergency situation report and set up a transport plan for an emergency patient according to an emergency situation or treatment environment in conjunction with a 119 report center or emergency situation management center.

The second server 120 may classify an emergency patient as either a first patient who requires a specific procedure or a second patient who does not require a specific procedure by using the level of severity obtained based on the severity classification used in common by emergency personnel and medical staff and the level of severity computed based on the prediction value. For example, the Prehospital Korean Triage and Acuity Scale (Pre-KTAS) method may be used as the severity classification method used in common by emergency personnel and medical staff.

**[Table 1]**

| [Comparisons between 119 Emergency Services Classification and KTAS Classification for Emergency Patients] | | | |
|---|---|---|---|
| 119 Emergency Services Classification | | KTAS Classification | |
| Emergency | Unstable vital signs, etc. | Level 1 | Resuscitation: cardiac arrest, critical illness, etc. |
| Near-emergency | In the case where treatment is required within several hours | Level 2 | Urgent: difficulty in breathing, vomiting, etc. |
| Potential Emergency | (Near -) emergency not applicable, Emergency room treatment required | Level 3 | Emergency: mild respiratory failure, etc. |
| Non - target | In the case of not emergency patient transport | Level 4 | Near-emergency: confusion, urinary tract infection, etc. |
| Death | In the case of an obvious sign or suspicion of death | Level 5 | Non - emergency: wound disinfection, prescription of medication, etc. |

As shown in Table 1, according to the Prehospital Korean Triage and Acuity Scale (Pre-KTAS) method, emergency patients are classified into grade 1 (resuscitation: very severe), grade 2 (urgent), grade 3 (emergency), grade 4 (near-emergency), and grade 5 (non-emergency: considerably mild) according to the severity of the emergency at the prehospital stage, and the medical treatment times for the respective grades are defined as immediate, within 10 minutes, within 30 minutes, within 60 minutes, and within 120 minutes. The Prehospital Korean Triage and Acuity Scale (Pre-KTAS) method indicates that as the classification grade decreases, the severity becomes higher, and thus, the priority for treatment becomes higher.

The second server 120 classifies an emergency patient as either a first patient who requires a specific procedure or a second patient who does not require a specific procedure by using the level of severity of the emergency patient measured by the severity classification method used in common by emergency personnel and medical staff and the level of severity computed based on the prediction value before transporting the emergency patient to a hospital. For example, the second server 120 may classify a patient who requires a procedure (e.g., a PCI procedure) that can only be performed at a specific hospital or by specific medical staff or a patient whose level of severity computed based on the prediction value is determined to be higher than a preset reference for each disease (e.g., a patient whose level of severity level is level 2 or higher based on the STEMI classification model) as the first patient.

Furthermore, the second server 120 may extract candidate hospitals for the treatment of an emergency patient from a group of hospitals matching the classification based on location and traffic information including at least one of the location of the emergency patient, real-time traffic conditions, and the number of ambulances heading to each hospital, may compute the degrees of suitability of the candidate hospitals by using capacity information including at least one of the emergency room congestion, emergency facility information, and medical staff information of each of the candidate hospitals, and may then set up a transport plan according to the order of priority of the hospitals based on the computed degrees of suitability.

Furthermore, the second server 120 may continuously determine the level of severity of an emergency patient transported from an emergency room by providing not only an alternative transport plan and transport for an emergency patient but also a monitoring service for a hospitalized patient, and may determine whether to transfer the patient to another hospital or transfer the patient from a general ward to an intensive care unit based on the determined level of severity.

FIG. 4 is a flowchart illustrating a method for providing a transport service for an emergency patient and the monitoring of a hospitalized patient based on electrocardiograms according to one embodiment of the present disclosure.

Referring to FIG. 4, the computing device 100 obtains the electrocardiogram data of an emergency patient at preset time intervals in response to an emergency situation report in step S10. In this case, the second server 120 may transmit a link, a QR code, or the like, via which electrocardiograms may be measured and transmitted, to the emergency patient. Once a connection using the link or QR code has been completed by the emergency patient, a guardian or a 119 emergency medical technician, the second server 120 may be connected with the local device 10, and the electrocardiograms of the emergency patient may be automatically measured and transmitted at periodic time intervals (for example, 3 minutes). Even when a periodic time interval for the measurement of an electrocardiogram has elapsed, the second server 120 may wait for electrocardiogram measurement for a predetermined period of time and then measure an electrocardiogram again after a predetermined period of time has elapsed if the emergency patient is moving or moves a lot. The reason for this is that the accuracy of the measured electrocardiogram decreases when the emergency patient is moving or moves a lot, and thus, the electrocardiogram may be determined to be unidentifiable when it is read.

The second server 120 transmits the obtained electrocardiogram data to the first server 110, and the first server 110 calculates a prediction value corresponding to the likelihood of occurrence of each of a plurality of heart diseases, such as myocardial infarction, cardiac arrest within 24 hours, arrhythmia, and/or the like, based on the electrocardiogram data by using the pre-trained neural network model in step S20.

The first server 110 transmits the generated prediction value to the second server 120, and the second server 120 determines the trend of changes in the prediction value transmitted at preset time intervals, computes the level of severity for the classification of an emergency patient based on the determined trend of changes and the characteristics of the plurality of diseases in step S30, and selects a target institution or facility suitable for the treatment of the emergency patient by using the computed level of severity in step S40.

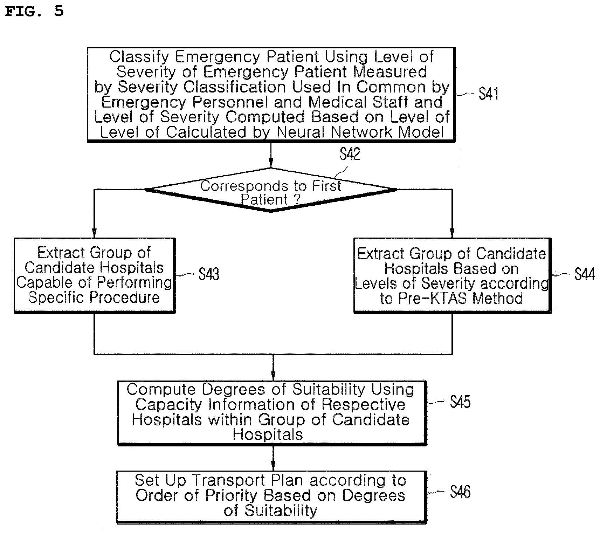

FIG. 5 is a flowchart illustrating in detail a method of selecting a target institution or facility suitable for the treatment of an emergency patient according to one embodiment of the present disclosure.

Referring to FIG. 5, the computing device 100 classifies an emergency patient as either a first patient who requires a specific procedure or a second patient who does not require a specific procedure by using the level of severity of the emergency patient measured by the severity classification method used in common by emergency personnel and medical staff and the level of severity computed based on the prediction value calculated by the neural network model in step S41.

In this case, the first patient may include at least one of a patient who requires a procedure that can only be performed at a specific hospital or by specific medical staff and a patient whose level of severity computed based on a prediction value is determined to be a preset level or higher for each disease.

In the case of the first patient, the computing device 100 extracts candidate hospitals for the treatment of the emergency patient from a hospital group matching the first patient based on location and traffic information including at least one of the location of the emergency patient, real-time traffic conditions, and the number of ambulances heading to each hospital capable of performing a specific procedure (e.g., a PCI procedure) in steps S42 and S43. In this case, the computing device 100, i.e., the second server 120, may collect real-time traffic information via the ITS National Traffic Information Center, and may collect the number of ambulances heading to the hospital via a navigation service operator that provides route guidance based on a user's real-time location information.

In the case of the second patient, the computing device 100 extracts candidate hospitals from a hospital group matching the second patient based on location and traffic information including at least one of the location of the emergency patient, real-time traffic conditions, and the number of ambulances heading to each hospital according to the level of severity based on the pre-KTAS in steps S42 and S44. The computing device 100 matches the second patient to a hospital group of regional/severe emergency centers when the level of severity according to the pre-KTAS corresponds to the first and second levels, matches the second patient to a hospital group of local emergency medical centers when the level of severity corresponds to the second and third levels, and matches the second patient to a hospital group of local emergency medical institutions when the level of severity corresponds to the fourth and fifth levels.

The computing device 100 may use location and traffic information to compute expected transport time information for the respective hospitals included in the hospital group matching the patient classification and extract candidate hospitals based on the expected transport time information.

The computing device 100 computes the degrees of suitability of the candidate hospitals by using capacity information including at least one of the emergency room congestion, emergency facility information, and medical staff information of each of the extracted candidate hospitals in step S45, and sets up a transport plan according to the order of priority of the hospitals based on the computed degrees of suitability in step S46. The second server 120 may periodically collect capacity information such as real-time congestion for each emergency room, emergency facility information, and medical staff information via the websites of the respective hospitals or the National Emergency Medical Information Network (NEDIS).

In this case, the computing device 100 may provide a communication service such as wired inquiry or voice AI inquiry by a paramedic in order to inquire of N hospitals, selected according to the order of priority of the hospitals, about whether to accept a patient when setting up a transport plan. After transporting an emergency patient to a suitable hospital according to the order of priority of the hospitals, the computing device 100 may transfer cumulative electrocardiogram data and analysis results (prediction values, the trends of changes, etc.) during the period from an emergency report to a final arrival at the hospital to medical staff in charge of treating the emergency patient. Furthermore, the computing device 100 may continuously determine the level of severity of an emergency patient even in a medical environment including an emergency room, and may continuously perform tasks such as a task of determining whether to transfer the patient to another hospital or transfer the patient from a general ward to an intensive care unit based on the determined level of severity, thereby providing a monitoring service for the emergency patient for a predetermined period of time or until the emergency patient's treatment is completed.

In this manner, in the present disclosure, electrocardiograms of an emergency patient are measured at periodic time intervals, a prediction value for the likelihood of occurrence of each of a plurality of heart diseases is computed using the pre-trained neural network model, and a transport plan for the emergency patient is set up based on the trend of changes in the prediction value for the likelihoods of occurrence of the heart diseases and the characteristics of the plurality of diseases. In this case, in the present disclosure, when the likelihood of occurrence of at least one heart disease is estimated to be low based on the change trend of the prediction value, the level of severity of the emergency patient is set to a preset level or lower. In contrast, when the likelihood of occurrence of at least one heart disease is estimated to be high, the level of severity of the emergency patient is determined to be considerably high, and thus a transport plan is set up such that the emergency patient can be rapidly transported to a hospital where a treatment or procedure for the heart disease can be performed.

Therefore, the present disclosure may provide a transport plan, such as a hospital where an emergency treatment and a specific procedure can be performed, a transport route, and/or the like, based on a fast and accurate prediction value for the likelihood of occurrence of a heart disease using the pre-trained neural network model, so that no unnecessary time is wasted in the rapid transport of an emergency patient and no resources or time are wasted in emergency treatment. Most importantly, the present disclosure may classify patients, such as patients having simple chest pain other than myocardial infarction and a patients having myocardial infarction, by using the neural network model, rather than a paramedic (or a 119 emergency medical technician), so that the appropriateness of emergency treatment according to emergency symptoms can be further increased in the pre-hospital stage of an emergency patient.

The various embodiments of the present disclosure described above may be combined with one or more additional embodiments, and may be changed within the range understandable to those skilled in the art in light of the above detailed description. The embodiments of the present disclosure should be understood as illustrative but not restrictive in all respects. For example, individual components described as unitary may be implemented in a distributed manner, and similarly, the components described as distributed may also be implemented in a combined form. Accordingly, all changes or modifications derived from the meanings and scopes of the claims of the present disclosure and their equivalents should be construed as being included in the scope of the present disclosure.

## Claims

1. A method for providing a transport service for an emergency patient and monitoring of a hospitalized patient based on electrocardiograms, the method being performed by a computing device including at least one processor, the method comprising:
obtaining electrocardiogram data of an emergency patient at preset time intervals;
calculating a prediction value corresponding to a likelihood of occurrence of each of a plurality of diseases based on the electrocardiogram data by using a pre-trained neural network model;
computing a level of severity for classification of the emergency patient based on the prediction value; and
selecting a target institution or facility suitable for treatment of the emergency patient by using the computed level of severity.

2. The method of claim 1, wherein computing the level of severity for the classification of the emergency patient based on the prediction value comprises identifying a trend of changes in the prediction value and determining the level of severity based on the identified trend of changes and characteristics of the plurality of diseases.

3. The method of claim 2, wherein identifying the trend of changes in the prediction value and determining the level of severity based on the identified trend of changes and the characteristics of the plurality of diseases comprises:
selecting one of determination methods for identifying the trend of changes in the prediction value according to the characteristics of the plurality of diseases; and
determining the level of severity based on the identified trend of changes identified according to the selected determination method.

4. The method of claim 3, wherein the determination method is any one of a method of determining whether the prediction value computed by the neural network model is in an upward trend, a method of determining whether the prediction value is in a downward trend or repeatedly falls and rises, a method of making a determination using a slope of a rising curve for the prediction value, a method of determining whether the prediction value deviates from a predetermined cutoff, and a method of determining whether the prediction value deviates from a predetermined size by marking the prediction value on a graph and then computing a size of an area.

5. The method of claim 1, wherein selecting the target institution or facility suitable for treatment of the emergency patient by using the computed level of severity comprises:
classifying the emergency patient as either a first patient who requires a specific procedure or a second patient who does not require a specific procedure by using a level of severity of the emergency patient measured by severity classification used in common by emergency personnel and medical staff and a level of severity computed based on the prediction value;
extracting candidate hospitals for treatment of the emergency patient from a group of hospitals matching the classification based on location and traffic information including at least one of a location of the emergency patient, real-time traffic conditions, and a number of ambulances heading to each hospital; and
computing degrees of suitability of the candidate hospitals by using capacity information including at least one of emergency room congestion, emergency facility information, and medical staff information of each of the candidate hospitals, and setting up a transport plan according to order of priority of the hospitals based on the computed degrees of suitability.

6. The method of claim 5, wherein the first patient includes at least one of a patient who requires a procedure that can only be performed at a specific hospital or by specific medical staff and a patient whose level of severity computed based on the prediction value is determined to be higher than a preset reference for each of the diseases.

7. The method of claim 5, wherein extracting the candidate hospitals for the treatment of the emergency patient from the group of hospitals matching the classification based on the location and traffic information including at least one of the location of the emergency patient, the real-time traffic conditions, and the number of ambulances heading to each hospital comprises:
computing expected transport time information for the hospitals included in the hospital group matching the classification; and
extracting candidate hospitals from the hospital group matching the classification based on the expected transport time information.

8. The method of claim 5, wherein computing the degrees of suitability of the candidate hospitals by using the capacity information including at least one of the emergency room congestion, emergency facility information, and medical staff information of each of the candidate hospitals, and setting up the transport plan according to the order of priority of the hospitals based on the computed degrees of suitability comprises providing a communication service in order to inquire of N hospitals, selected according to the order of priority of the hospitals, about whether to accept the patient.

9. A computer program stored in a computer-readable storage medium, the computer program performing operations for providing a transport service for an emergency patient and monitoring of a hospitalized patient based on electrocardiograms when executed on one or more processors, wherein the operations comprises operations of:
obtaining electrocardiogram data of an emergency patient at preset time intervals;
calculating a prediction value corresponding to a likelihood of occurrence of each of a plurality of diseases based on the electrocardiogram data by using a pre-trained neural network model;
computing a level of severity for classification of the emergency patient based on the prediction value; and
selecting a target institution or facility suitable for treatment of the emergency patient by using the computed level of severity.

10. A computing device for providing a transport service for an emergency patient and monitoring of a hospitalized patient based on electrocardiograms, the computing device comprising:
a processor including at least one core; and
memory including program codes executable on the processor;
wherein the processor, according to execution of the program codes,
obtains electrocardiogram data of an emergency patient at preset time intervals;
calculates a prediction value corresponding to a likelihood of occurrence of each of a plurality of diseases based on the electrocardiogram data by using a pre-trained neural network model;
computes a level of severity for classification of the emergency patient based on the prediction value; and
selects a target institution or facility suitable for treatment of the emergency patient by using the computed level of severity.
